# EUROPEAN PATENT SPECIFICATION

(11) **EP 1 317 222 B1**
(45) Date of publication and mention of the grant of the patent: **05.04.2006**
(21) Application number: 01976608.8
(22) Date of filing: 13.09.2001
(51) Int. Cl.: A61C 13/00, A61K 6/04, B22F 3/12

(54) **METHOD FOR THE PREPARATION OF DENTAL PROSTHETIC ARTEFACTS BY MEANS OF POWDER SINTERING**
VERFAHREN ZUR HERSTELLUNG VON DENTALEN PROTHESEN DURCH SINTERN VON PULVER
PROCEDE D'ELABORATION DE PRODUITS DE PROTHESES DENTAIRES PAR FRITTAGE

(30) Priority: 13.09.2000 IT AL000007
(43) Date of publication of application: 11.06.2003
(73) Proprietor: Nobil Metal S.p.A., I-10138 Torino (IT)
(72) Inventor: VENTURINI, Daniele, I-14018 Villafranca d'Asti (IT); WALTHER VENTURINI, Giuseppe, I-14018 Villafranca d'Asti (IT)
(74) Representative: Leone, Mario
(86) International application number: PCT/IT2001/000476
(87) International publication number: WO 2002/022041

(56) References cited:
- WO-A-01/50975
- US-A- 5 234 343
- US-A- 6 027 012

## Description

The present invention relates to a method for obtaining artefacts or metal structures for odontotechny and dentistry, providing for the use of powders of the metals and of the alloys commonly used in the practice of odontotechnical laboratories.

The processing of metals and alloys is a widely followed practice in the operations that take place in an odontotechnical laboratory. Such work processes entail a wide range of different operations- and, for instance, the production - with the so-called lost wax method - of metallic dental structure for prosthetic uses, as a consequence of the loss of one or more teeth because of pathological or traumatic events.

As it is well known to the persons skilled in the art, the lost wax technique consists of numerous successive operations starting with the dentist's taking the impression of the patient's teeth. From this impression, a cast of the teeth is obtained, with the aid of which the dentist builds a wax model of the prosthetic component (crown, bridge or other) to be made of metal. This model is incorporated in a refractory material known as coating and which, solidifying, encloses the model in a thermally stable shell. A subsequent heat treatment allows to eliminate, through appropriate vents, the wax part and to obtain a case having in its interior a cavity with the shape of the original wax model. This cavity is filled with the molten metal casting and, after the mechanical elimination of the coating, the metallic prosthetic component is obtained, to be used as it is or coated, for aesthetic purposes, with ceramic or resin.

The lost wax technique allows to obtain good results, but - as the short description provided above shows - it consists of a long process, not without its difficulties.

Other dentistry work processes that use metals are, for instance, welding operations, repairs of broken or damaged artefacts, correcting or reheating a component that is not wholly satisfactory, and the like. Such work processes, mentioned as examples of a wider range of operations, require additional metallic weld material. In particular, the addition of further metal necessarily requires melting said metal. This operation can cause a deformation of the existing artefact, due to the need to reach high temperatures. The attempt to avoid this drawback using different weld alloys, in particular alloys with a lower melting point than that of the metal of the existing artefact, is also not free of drawbacks. The presence of different metals in mutual electrical contact can lead, in the oral environment, to the onset of corrosion phenomena, with the consequent deterioration of mechanical properties, aesthetically displeasing discoloration and the release of ions in the oral cavity of the person wearing the prosthesis.

From the above, it is readily apparent that all the operation that provide for the realisation, modification, correction and re-adaptation of a prosthetic artefact through the use or addition of metallic material present quite a few practical and constructional difficulties. For this reason, different alternative methods have been proposed; among them, particularly promising appear the processes that provide for sintering metallic powders. The consolidation of metallic powders by sintering is a technique with widespread industrial use and it is described, for instance, in the book by C.G. Goetzel "Treatise on Powder Metallurgy" published by Interscience Publishers Ltd., London, 1949.

The application of powder metallurgy to the practice of dentistry is described, for instance, in US Patent 4,689,197, which teaches the realisation of dental prostheses by means of sintering metallic powders, optionally with the use of glass or ceramic powders. According to said patent, the metallic powders, made plastic with water, are adapted and conformed to a prepared model of the prosthetic component to be obtained, coated with a thin layer of wax that is eliminated before sintering. Another US Patent, 4,828,495 describes the realisation of metallic prostheses by sintering a paste obtained from metal powders with an appropriate organic binder. The paste is modelled on a prepared component, wherefrom it is isolated by means of another metallic layer, also obtained by sintering. The powder sintering technique is also used according to US Patent 4,980,124, which provides for the metallic powders to be sintered on a thin sheet of palladium or suitable alloys, adapted to a model of the component to be realised, in such a way as to obtain a metallic structure that is difficult to deform, whereon a ceramic crown is constructed.

US Patent 5,234,343 discloses a method and a material that allow to obtain prosthetic components starting from metallic powders constituted by a metal with high melting point and a metal with low melting point.

The technique of sintering metallic powders, as taught in the aforesaid patents, allows to obtain metallic prostheses in a faster manner, with fewer scraps and with greater dimensional accuracy relative to the lost wax technique. However, there are still some problems that limit the practical and complete attainment of these advantages. First of all, not all the aforesaid known techniques allow to complete all of the operations listed above. Moreover, the need successfully to perform the sintering process entails come drawbacks, such as the use - often suggested - of furnaces able to reach high vacuum conditions. This requirement is imposed by the need to sinter powders suitable for the metal-ceramic technique. As is well known to those versed in the art, such alloys contain - in addition to a high percentage of noble metals - also a certain quantity of non noble and easily oxidised metals. The latter make the sintering operation more difficult and not achievable under atmospheric pressure conditions or with the degree of vacuum reachable in common ceramic furnaces.

For example, according to the aforesaid US Patent 5,234,343, sintering is performed at atmospheric pressure, but this leads to the need to use powders with high noble metal content. This in turn causes high raw material costs and entails the impossibility to form chemical bonds between metal and ceramic, so that adhesion between these two components is left only to mechanical action.

Since the establishment of a strong bond between metal and ceramic is an essential condition for the successful outcome of the work, the lack of a chemical bond is a non negligible limitation.

Moreover, Italian Patent No. 1271360 in the name of the same Applicant describes a method for obtaining artefacts starting from alloy powders for metal-ceramic at ambient pressure, providing for the use of graphite containers, under which the sintering process takes place. The need to effect the sintering under graphite containers constitutes a limitation to the application of the technique, for the following two reasons.

First of all, the dimension of the work that can be executed is limited by the dimensions of the graphite container. In the second place, the introduction of graphite objects into a ceramic furnace is not viewed favourably by the user, due to the possibility of contamination from the carbon of the graphite containers. It is well known that the carbon may combine with the palladium contained in many alloys used for dentistry, giving rise to compounds that make the alloy considerably more fragile, to the point that it is no longer suitable for normal conditions of use. Moreover, carbon contamination may originate bubbles when the ceramic is cooked and it may promote the formation of discoloration and aesthetic flaws in the ceramic.

Further, from US-A-6 027 012 it is known a method for the sintering of a metal sheet in dental prostheses made of a metal powder after a modelling step of the same directly on to the master mould, therefore achieving the suppression of the adjustment step, i.e. namely scrapping. However this method has a drawback given to the fact that i t further provides the step of detaching the sheet of metal powder from the master model in order to coat the internal part of the said metal sheet with a pasty mixture of refractory powder product. This further step entails a further cost related to time working process.

Taking the above into account, the aim of the present invention is to find a method that allows to achieve, in an easy, precise and economical manner, the production of metallic dental structures for prosthetic uses and it simultaneously enables also to achieve the addition of metallic material for modification, correction, reinforcement or junction purposes, or for any other requirements, eliminating the drawbacks of the aforesaid known methods and above all the need to use vacuum conditions or particular atmospheres as well as graphite containers, and determining a considerable improvement of the work processes used thus far, while it allows fully to exploit the advantages of sintering and to obtain prosthetic structures and to realise additions and corrections in a more convenient manner relative to the prior art.

Moreover, the present invention entails the considerable advantage and the peculiarity of working directly on the master model and not on the refractory material, thereby avoiding the duplication step, with consequent time savings and reduced risk of imprecision.

Moreover, the present invention provides for the use of common dental alloys such as gold or pure silver.

Hence, the present invention provides a method for obtaining prosthetic metallic structures, corrections, additions, adaptations of details, and the like, as claimed in claim 1.

According to a preferred characteristic of the invention, an organic liquid is used for the refractory coating material, whose molecule contains 1 to 10 carbon atoms, preferably from 1 to 5 carbon atoms such as, for instance, methyl alcohol, ethyl alcohol, propyl alcohol, acetone, ethyl-methyl-ketone, diethyl-ketone, acrylic acid, metacrylic acid, diethanolamine, diethylamine, and the like. Mixtures of two or more of said liquids can also be used.

Next to carbon, the molecule of the organic liquid used according to the invention to mix the coating material, can also contain other elements as well, such as nitrogen, oxygen, sulphur, chlorine, fluorine and all elements that commonly bind with carbon.

The organic liquid and the coating material react to each other at the time of mixing and/or inside the furnace, to generate conditions suitable for sintering, strengthening also the normal protection against contact with the atmosphere, which protection is determined by the protective shell constituted by the coating material.

In a particularly favourable embodiment of the present invention, a prosthetic artefact is obtained in the following way.

The material for obtaining the prosthesis is constituted by a metallic powder which has the same composition as the alloys and the pure metals commonly used in dentistry. This powder is employed as such or it is preferably incorporated in an additive that facilitates its deposition and/or modelling. Moreover, a diluting agent can be used to make the paste even more malleable and workable. Typical, but not limiting, examples of additives are constituted by polyethylene, glycol, glycerine, butylic, isobutylic or natural rubber, natural or synthetic wax, as well as mixtures of one or more of said substances. Typical, but not limiting, examples of diluting agents are constituted by water, hydrocarbons, alcohols, poly-oils and mixtures thereof.

The size of the particles of the powders used normally ranges from 0.1 and 1000 µm. Powder composition is not critical and may correspond to the compositions used in common practice with traditional methods.

According to an embodiment of the present invention, a component of a prosthesis for the metal-ceramic technique can be obtained starting from powders with identical composition to that of the alloy used normally for the same purposes.

The metallic powder mixture is worked in such a way as to obtain plates of defined thickness. Indicative, but not limiting, values of the thickness of such plates range between 0.15 and 0.40 millimetres. In a single plate, the metallic powder may be constituted by pure metal, alloy, or a mixture of pure metal/pure metal or pure metal/alloy or alloy/alloy with a volumetric ratio ranging between 0.5 and 1.5 and preferably equal to 1.

The plate (or a part thereof) is adapted directly to the plaster model of the component to be obtained. After adapting and modelling this plate, it is coated with a refractory coating material according to the invention, i.e. mixed with an organic liquid, as described above.

After some minutes, the modelled part is extracted from the plaster model and completely coated with metallic coating as per the present invention. The whole set is then moved to a common dentistry furnace, where sintering is performed. It is preferable, but not binding, to keep the prepared item, before sintering, for a certain time interval to a temperature lower than sintering temperature, to obtain the elimination of the excess organic liquid.

Sintering is performed in a ceramic or pre-heating furnace, at ambient pressure and in normal atmosphere, at a temperature equal to about 80 % of the melting temperature of the alloy. The metallic artefact thus produced can be ceramic coated and worked as usual.

The numerous advantages of the invention are readily apparent to those skilled in the art. The manufacture of metallic prosthetic structures such as crowns, bridges and the like, as well as their correction, welding and re-modelling, are considerably facilitated by the method according to the invention which allows fully to exploit the advantages of the powder sintering technique and does not entail any additional equipment complexity or contamination risks for the materials.

In addition to the manufacture of prostheses, the method according to the present invention is particularly advantageous for effecting modifications, corrections of the biting surface, corrections of the general shape of said prostheses, as well as welding different components in order to obtain a single body. In such cases, the welding material (which preferably has the same composition of the alloy constituting the prosthetic artefact) is deposited with extreme precision and ease, thanks to the ease of manipulation of the metal-additive mix. The component to be sintered is enclosed in the coating material mixed, according to the invention, with the organic liquid. The method according to the present invention, moreover, allows the supermelting technique using suitable alloys.

Moreover, the elements produced by sintering the metal according to the method of the present invention, can thereafter be united and/or modified with:
- the addition of more material suitable for sintering,
- traditional welding materials
- supermelting (lost wax method).

Hereafter, some embodiment examples of the method according to the invention are provided purely by way of illustrative and not limiting example.

### Example 1

A metal crown was manufactured using a quantity of New Ceramit USA 88 alloy (Nobil Metal, Villafranca, AT) with wax added (Industria Zingardi, Novi Ligure, AL). The composition of the alloy, as declared by the manufacturer, is as follows:

| Au | Pd | Cu | Ga | Ru |
|---|---|---|---|---|
| 2.0 % | 79.0 % | 9.9 % | 9.0 % | 0.1 % |

The mixture was subjected to pressure to increase the consistency of the material, obtaining a plate with a thickness of 0.30 mm. A portion cut off from said plate was wound about a plaster model and then pressed, to adapt it to the shape of the model.

At the end of the adapting process, the metal paste was coated with coating material (Sintercast RDM, Nobil Metal). The coating material used is characterised by the following composition:

| | |
|---|---|
| Crystalline silica (quartz) | 10-30 % |
| Crystalline silica (cristobalite) | 40-70 % |
| Phosphate-based powders | 10-30 % |
| Metal oxides | 3-15 % |

Four samples (a), (b), (c) and (d) were prepared, each obtained with the same coating material but mixed each time with one of the following liquids:
a) The liquid normally used for mixing the coating (Sintercast RDM liquid, with aqueous base and 15-40 % amorphous colloidal silica).
b) Aqua
c) Denatured ethyl alcohol
d) Acetone

The items were sintered in a ceramic furnace (of the kind commercially known as LECTRA, Nobil Metal, Villafranca, AT) at 1050 °C for 10 minutes.

The results of the sintering process were assessed through the visual observation of the products and by means of mechanical stressing. In particular, the items were blocked by means of clamps and subjected to bending with pincers. The products were classified according to a scale from 1 to 5, where "1" means the product underwent no compacting, whereas "5" means the product has the suitable consistency for its intended use. The test was repeated three times for each series of four samples ("a" through "d") and the results, expressed as the mean of the observations, are set out in the following table:

| Sample | Result |
|---|---|
| a | 1 |
| b | 1 |
| c | 5 |
| d | 5 |

### Example 2

The experiment of Example 1 was repeated with powders of the Pal Keramit (Nobil Metal, Villafranca, AT) alloy. The composition of the alloy, as declared by the manufacturer, is as follows:

| Ag | Pd | Sn | Ga | In | Ru |
|---|---|---|---|---|---|
| 32.0 % | 57.5 % | 8.0 % | 1.5 % | 1.0 % | 0.1 % |

The sintering process was conducted for 10 minutes at 1010 °C. Then, the assessment system described in the previous example was used for the same number of samples and the following results were obtained:

| Sample | Result |
|---|---|
| a | 1 |
| b | 1 |
| c | 5 |
| d | 5 |

### Example 3

The experiment of Examples 1 and 2 was repeated using powders of a yellow alloy having the following composition:

| Au | Pt | Sn | In | Ir | Ru |
|---|---|---|---|---|---|
| 80.6 % | 15.4 % | 1.6 % | 1.4 % | 0.4 % | 0.6 % |

The sintering process was conducted for 15 minutes at 990 °C, then the same assessment system described in Example 1 was used and the following results were obtained:

| Sample | Result |
|---|---|
| a | 1 |
| b | 1 |
| c | 5 |
| d | 5 |

### Example 4

Using the alloy of sample 1, three separate units were obtained, destined to a three-element bridge, constituted by two crowns and by an intermediate element. On the junction points, a paste obtained from the alloy of Example 1 with wax added was delicately modelled. The elements were then brought in mutual contact. Four samples a), b), c) and d) were prepared with refractory coating material mixed each time with one of the liquids of Example 1. The sintering process was conducted in the conditions of Example 1. The finished products do not reveal any trace of added material at the junction points and perfectly adapt to the model of the stumps and are suitable to prosthetic use in the case of samples c) and d), whereas sintering is not satisfactory in the case of samples a) and b).

### Example 5

A metal crown was obtained with the lost wax technique, using the Pal Keramit alloy as a model. The thickness of the part destined to cover the stump was measured with a micrometer and a maximum value of 0.8 mm was recorded. To assess the effectiveness of the method according to the invention to obtain thickness increases, the item was uniformly coated with a plate of Pal Keramit alloy powder, with wax added. The sintering process was conducted with the times and in the ways of cases b) and d) of Example 2. Thickness was measured again and a uniform increase of 0.5 mm was recorded in case d), whilst the added powder was not found to have been sintered in case b) .

### Example 6

A mixture of pure gold powder was obtained by combining gold powder and wax in the proportions of 3.5 g of wax per 100 g of gold. The mixture thus obtained was pressed to obtain a platelet with a thickness of 0.30 mm. A cut-off portion of said plate was wound about a plaster model and then pressed, to adapt it to the shape of the model.

At the end of the adaptation process, the metal paste was coated with coating material (Sintercast RDM, Nobil Metal) mixed with acetone. The items were sintered in a ceramic furnace (LECTRA, Nobil Metal, Villafranca, AT) a 1030 °C for 4 minutes.

The results of the sintering process were assessed through the visual observation of the product and by means of mechanical stressing, as described in Example 1. The result obtained, according to the scale described in Example 1, was assigned a value of 5.

### Example 7

A mixture of pure silver powder was obtained by combining silver powder and wax in the proportions of 6.5 g of wax per 100 g of silver. The mixture thus obtained was worked to obtain a platelet with a thickness of 0.30 mm. A cut-off portion of this platelet is wound about a plaster model and then pressed, to adapt it to the shape of the model.

At the end of the adaptation process, the metallic paste was coated with coating material (Sintercast RDM, Nobil Metal) mixed with acetone. The items were sintered in a ceramic furnace (LECTRA, Nobil Metal, Villafranca, AT) at 920 °C for 4 minutes*.*

The results of the sintering process were assessed through the visual observation of the product and by means of mechanical stressing, as described in Example 1. The result obtain, according to the scale described in Example 1, was assigned a value of 5.

### Example 8

A plate with a thickness of 0.35 mm was obtained by homogeneously mixing pure gold powder and pure silver powder. The final plate had a volumetric ratio of 1 to 1. A portion of said plate was wound about a plaster model and the pressed, to adapt it to the shape of the model. At the end of the adaptation process, the metal paste was coated with coating material (Sintercast RDM, Nobil Metal) mixed with acetone. The items were sintered in a ceramic furnace (LECTRA, Nobil Metal, Villafranca, AT) at 990 °C for 4 minutes.

The results of the sintering process were assessed through the visual observation of the product and by means of mechanical stressing, as described in Example 1. The result obtained, according to the scale described in Example 1, was assigned a value of 5.

### Example 9

A plate with a thickness of 0.34 mm was obtained mm by homogeneously mixing pure gold powder and powder of an alloy with high gold content (Au +PGM = 100 %) . A portion of the plate was wound about a plaster model and then pressed, to adapt it to the shape of the model. At the end of the adapting process, the metal plate was coated with coating material (Sintercast RDM, Nobil Metal) mixed with acetone. The items were sintered in a ceramic furnace (LECTRA, Nobil Metal, Villafranca, AT) at 1086 °C for 8 minutes.

The results of the sintering process were assessed through the visual observation of the product and by means of mechanical stressing, as described in Example 1. The result obtained, according to the scale described in Example 1, was assigned a value of 5.

### Example 10

A plate with a thickness of 0.36 mm was obtained by homogeneously mixing pure gold powder and powder of an alloy with high gold content (Au +PGM = 97.8 %). A portion of the plate was wound about a plaster model and then pressed, to adapt it to the shape of the model. At the end of the adapting process, the metal plate was coated with coating material (SINTERCAST RDM, Nobil Metal) mixed with acetone. The items were sintered in a ceramic furnace (LECTRA, Nobil Metal, Villafranca, AT) at 1075 °C for 8 minutes.

The results of the sintering process were assessed through the visual observation of the product and by means of mechanical stressing, as described in Example 1. The result obtained, according to the scale described in Example 1, was assigned a value of 5.

### Example 11

A plate with a thickness of 0.33 mm was obtained by homogeneously mixing pure gold powder and powder of an alloy characterised by a content of Au +PGM of 84.0 %. A portion of the plate was wound about a plaster model and then pressed, to adapt it to the shape of the model. At the end of the adapting process, the metal plate was coated with coating material (SINTERCAST RDM, Nobil Metal) mixed with acetone. The items were sintered in a ceramic furnace (LECTRA, Nobil Metal, Villafranca, AT) at 1080 °C for 8 minutes.

The results of the sintering process were assessed through the visual observation of the product and by means of mechanical stressing, as described in Example 1. The result obtained, according to the scale described in Example 1, was assigned a value of 5.

### Example 12

A plate with a thickness of 0.35 mm was obtained by homogeneously mixing pure silver powder and powder of an alloy with high gold content (Au +PGM = 97.8 %). A portion of the plate was wound about a plaster model and then pressed, to adapt it to the shape of the model. At the end of the adapting process, the metal plate was coated with coating material (SINTERCAST RDM, Nobil Metal) mixed with acetone. The items were sintered in a ceramic furnace (LECTRA, Nobil Metal, Villafranca, AT) at 1000 °C for 8 minutes.

The results of the sintering process were assessed through the visual observation of the product and by means of mechanical stressing, as described in Example 1. The result obtained, according to the scale described in Example 1, was assigned a value of 5.

### Example 13

A plate with a thickness of 0.35 mm was obtained by homogeneously mixing powder of an alloy with high gold content (Au + PGM = 100 %) and powder of an alloy characterised by Au +PGM = 97.8 %. A portion of the plate was wound about a plaster model and then pressed, to adapt it to the shape of the model. At the end of the adapting process, the metal plate was coated with coating material (Sintercast RDM, Nobil Metal) mixed with acetone. The items were sintered in a ceramic furnace (LECTRA, Nobil Metal, Villafranca, AT) at 1095 °C for 8 minutes.

The results of the sintering process were assessed through the visual observation of the product and by means of mechanical stressing, as described in Example 1. The result obtained, according to the scale described in Example 1, was assigned a value of 5.

### Example 14

A series of prosthetic elements obtained as described in the previous examples (1→13) was welded using portions of a plate having the same chemical composition as the prosthetic element. The metallic paste was adapted in the point of interest joining two single crowns, then proceeding with the sintering process in a ceramic furnace (LECTRA, Nobil Metal, Villafranca, AT) with the same parameters used for sintering the initial artefact, except for the sintering temperature, lower by 5 - 10 °C than the initial one. The results of the sintering process were assessed through the visual observation of the product and by means of mechanical stressing, as described in Example 1. The result obtained, according to the scale described in Example 1, was assigned a value of 5.

### Example 15

A series of prosthetic elements obtained as described in Examples 1→13 was modified adding and modelling portions of a plate having the same chemical composition as the prosthetic element. The metallic paste was added in the area of interest, modifying the anatomic profile of the prosthesis and then proceeding with the sintering process in a ceramic furnace (LECTRA, Nobil Metal, Villafranca, AT) with the same parameters used for sintering the initial artefact, with the exception of the sintering temperature, 5 - 10 °C lower than the usual one. The results of the sintering process were assessed through the visual observation of the product and by means of mechanical stressing, as described in Example 1. The result obtained, according to the scale described in Example 1, was assigned a value of 5.

### Example 16

A series of prosthetic elements obtained as described in Examples 1→13 was welded using the classic blowpipe welding method. Two single crowns, after incorporation in the refractory material and subsequent pre-heating in the furnace were welded with the aid of the blowpipe, according to the technique widely known as blowpipe welding, with appropriate welding material. The success of the welding process was assessed through the visual observation of the product and by means of mechanical stressing, as described in Example 1. The result obtained, according to the scale described in Example 1, was assigned a value of 5.

### Example 17

A series of prosthetic elements obtained as described in Examples 1→13 was modified adding metal (welding material) with the traditional blowpipe welding technique. The welding material used, characterised by a working temperature that is 50 °C lower than the sintering temperature, flowed perfectly on the surface to be modified, thereby demonstrating an excellent ability to wet said surface. The results of the sintering process were assessed through the visual observation of the product and by means of mechanical stressing, as described in Example 1. The result obtained, according to the scale described in Example 1, was assigned a value of 5.

### Example 18

A series of prosthetic elements obtained as described in Examples 1→13 was joined using the supermelting technique (lost wax method). Two crowns were joined modelling an intermediate element with wax. The bridge structure thereby obtained (crowns plus wax structure) was then placed in a smelting coating (Proper Vest Fast Nobil Metal, AT) and incorporated in a smelting cylinder. The pre-heating and smelting operations were then carried out, using an alloy having a suitable casting temperature (Keramit Bio Uno Nobil Metal), close to the sintering temperature. The success of the supermelting process was assessed through the visual observation of the product and by means of mechanical stressing, as described in Example 1. The result obtained, according to the scale described in Example 1, was assigned a value of 5.

### Example 19

A series of prosthetic elements obtained as described in Examples 1→13 was modified adding metal with the traditional lost wax technique. Some crowns were modelled adding wax and thereby modifying the anatomical profile. The structure thus obtained (crown plus wax addition) was then placed in a smelting coating (Proper Vest Fast Nobil Metal) and incorporated in a smelting cylinder. The pre-heating and smelting processes were then performed, using an alloy with a suitable casting temperature (Keramit Bio Uno Nobil Metal), close to the sintering temperature. The success of the supermelting process was assessed through the visual observation of the product and by means of mechanical stressing, as described in Example 1. The result obtained, according to the scale described in Example 1, was assigned a value of 5.

## Claims

1. A method for obtaining dental prosthetic structures and the like, comprising the steps of adapting a plate made of metal powder with organic compounds added to a model made of plaster or the like; modelling said plate adapted on said model made of plaster or the like, and final sintering of the prepared item,
**characterised in that** it further comprises the step of directly coating the outer surface of the prepared item with a mixture of a refractory coating material with an organic liquid before the sintering step.

2. A method according to claim 1, wherein said metallic powder is a powder of pure metal.

3. A method according to claim 1, wherein said metallic powder is a powder of metallic alloys.

4. A method according to claim 1, **characterised in that** said plate is obtained by mixing different metallic powders.

5. A method according to claim 1, **characterised in that** said plate is obtained by mixing at least two metallic powders of pure metal.

6. A method according to claim 1, **characterised in that** said plate is obtained by mixing at least two metallic powders of pure metal and metal alloy.

7. A method according to claim 1, **characterised in that** said plate is obtained by mixing at least two metallic powders of metal alloy.

8. A Method according to any of the previous claims from 1 to 7, wherein said mixing of metallic powders has a volumetric ratio between the components that ranges between 0.5 and 1.5.

9. A method according to in the previous claim, wherein said volumetric ratio is equal to 1.

10. A method according to any of the previous claims, **characterised in that** said organic liquid contains a substance comprising from 1 to 10 carbon atoms.

11. A method according to the previous claim, **characterised in that** the organic liquid is selected from the following compounds: ethyl alcohol, acetone, methyl alcohol, propyl alcohol, ethyl-methyl-ketone, diethyl-ketone, acrylic acid, metacrylic acid, diethanolamine, diethylamine, and the like, and mixtures of two or more of said substances.

12. A method according to one or more of the previous claims, **characterised in that** any excess organic liquid is eliminated by means of permanence in the furnace at a temperature lower than sintering temperature.

13. A method according to one or more of the previous claims, **characterised in that** said metallic powder is constituted by an alloy containing at least 50 % of Pd.

14. A method according to one or more of the previous claims 1 through 12, **characterised in that** the metallic powder is constituted by an alloy containing at least 50 % of Au.

15. A method according to one or more of the previous claims 1 through 12, **characterised in that** the metallic powder is constituted by an alloy containing at least 50 % of Ag.

16. A method according to one or more of the previous claims 1 through 12, **characterised in that** the metallic powder is constituted by pure gold.

17. A method according to one or more of the previous claims 1 through 12, **characterised in that** the metallic powder is constituted by pure silver.

18. A method according to one or more of the previous claims, **characterised in that** said additive for said metallic powder is an organic compound selected from the following compounds:
Polyethylene glycol, glycerine, butylic rubber, isobutylic rubber, natural rubber, natural wax, synthetic wax, and the like and mixtures of two or more of said substances.

19. A method according to one or more of the previous claims, **characterised in that** the dimensions of the particles of the powders used normally range between 0.1 and 1000 µm.

## Patentansprüche

1. Verfahren zum Herstellen von Dentalprothesegebilden und dgl., beinhaltend die Schritte Anpassen eines Belages, der aus Metallpulver mit zugesetzten organischen Verbindungen besteht, an ein Modell, das aus Gips od. dgl. besteht; Modellieren des Belages, der an das Modell angepasst ist, das aus Gips od. dgl. besteht, und schließlich Sintern des hergestellten Gegenstands,
**dadurch gekennzeichnet, dass** es weiter den Schritt beinhaltet direktes Überziehen der äußeren Oberfläche des hergestellten Gegenstands mit einer Mischung aus einem feuerfesten Überzugsmaterial und einer organischen Flüssigkeit vor dem Sinterschritt.

2. Verfahren nach Anspruch 1, wobei das Metallpulver ein Pulver aus reinem Metall ist.

3. Verfahren nach Anspruch 1, wobei das Metallpulver ein Pulver aus Metalllegierungen ist.

4. Verfahren nach Anspruch 1, **dadurch gekennzeichnet, dass** der Belag durch Mischen von verschiedenen Metallpulvern hergestellt wird.

5. Verfahren nach Anspruch 1, **dadurch gekennzeichnet, dass** der Belag durch Mischen von wenigstens zwei Metallpulvern aus reinem Metall hergestellt wird.

6. Verfahren nach Anspruch 1, **dadurch gekennzeichnet, dass** der Belag durch Mischen von wenigstens zwei Metallpulvern aus reinem Metall und Metalllegierung hergestellt wird.

7. Verfahren nach Anspruch 1, **dadurch gekennzeichnet, dass** der Belag durch Mischen von wenigstens zwei Metallpulvern aus Metalllegierung hergestellt wird.

8. Verfahren nach einem der vorhergehenden Ansprüche 1 bis 7, wobei die Mischung mit Metallpulvern ein Volumenverhältnis zwischen den Komponenten hat, das in einem Bereich zwischen 0,5 und 1,5 liegt.

9. Verfahren nach dem vorhergehenden Anspruch, wobei das Volumenverhältnis gleich 1 ist.

10. Verfahren nach einem der vorhergehenden Ansprüche, **dadurch gekennzeichnet, dass** die organische Flüssigkeit einen Stoff enthält, der 1 bis 10 Kohlenstoffatome umfasst.

11. Verfahren nach dem vorhergehenden Anspruch, **dadurch gekennzeichnet, dass** die organische Flüssigkeit aus den folgenden Verbindungen ausgewählt wird: Ethylalkohol, Aceton, Methylalkohol, Propylalkohol, Ethylmethylketon, Diethylketon, Acrylsäure, Metacrylsäure, Diethanolamin, Diethylamin und dgl. und Gemischen von zwei oder mehr als zwei dieser Stoffe.

12. Verfahren nach einem oder mehreren der vorhergehenden Ansprüche, **dadurch gekennzeichnet, dass** jedwede überschüssige organische Flüssigkeit eliminiert wird mit Hilfe von Permanenz im Ofen bei einer Temperatur unterhalb der Sintertemperatur.

13. Verfahren nach einem oder mehreren der vorhergehenden Ansprüche, **dadurch gekennzeichnet, dass** das Metallpulver durch eine Legierung gebildet wird, die wenigstens 50 % Pd enthält.

14. Verfahren nach einem oder mehreren der vorhergehenden Ansprüche 1 bis 12, **dadurch gekennzeichnet, dass** das Metallpulver durch eine Legierung gebildet wird, die wenigstens 50 % Au enthält.

15. Verfahren nach einem oder mehreren der vorhergehenden Ansprüche 1 bis 12, **dadurch gekennzeichnet, dass** das Metallpulver durch eine Legierung gebildet wird, die wenigstens 50 % Ag enthält.

16. Verfahren nach einem oder mehreren der vorhergehenden Ansprüche 1 bis 12, **dadurch gekennzeichnet, dass** das Metallpulver durch reines Gold gebildet wird.

17. Verfahren nach einem oder mehreren der vorhergehenden Ansprüche 1 bis 12, **dadurch gekennzeichnet, dass** das Metallpulver durch reines Silber gebildet wird.

18. Verfahren nach einem oder mehreren der vorhergehenden Ansprüche, **dadurch gekennzeichnet, dass** der Zusatz für das Metallpulver eine organische Verbindung ist, die aus den folgenden Verbindungen ausgewählt wird:
Polyethylenglycol, Glycerin, Butylgummi, Isobutylgummi, natürlicher Gummi, natürliches Wachs, synthetisches Wachs und dgl. und Gemischen von zwei oder mehr als zwei dieser Stoffe.

19. Verfahren nach einem oder mehreren der vorhergehenden Ansprüche, **dadurch gekennzeichnet, dass** die Abmessungen der Partikel der Pulver, die normalerweise verwendet werden, in einem Bereich zwischen 0,1 und 1000 µm liegen.

## Revendications

1. Procédé pour obtenir des structures de prothèse dentaire et analogue, comportant les étapes consistant à adapter une plaque constituée d'une poudre métallique avec des composés organiques ajoutés à un modèle constitué de plâtre ou analogue, modéliser ladite plaque adaptée sur ledit modèle constitué de plâtre ou analogue, et fritter en fin de compte l'article préparé,
**caractérisé en ce qu'**il comporte de plus l'étape consistant à revêtir directement la surface extérieure de l'article préparé à l'aide d'un mélange d'un matériau de revêtement réfractaire et d'un liquide organique avant l'étape de frittage.

2. Procédé selon la revendication 1, dans lequel ladite poudre métallique est une poudre de métal pur.

3. Procédé selon la revendication 1, dans lequel ladite poudre métallique est une poudre d'alliages métalliques.

4. Procédé selon la revendication 1, **caractérisé en ce que** ladite plaque est obtenue en mélangeant différentes poudres métalliques.

5. Procédé selon la revendication 1, **caractérisé en ce que** ladite plaque est obtenue en mélangeant au moins deux poudres métalliques de métal pur.

6. Procédé selon la revendication 1, **caractérisé en ce que** ladite plaque est obtenue en mélangeant au moins deux poudres métalliques de métal pur et d'alliage métallique.

7. Procédé selon la revendication 1, **caractérisé en ce que** ladite plaque est obtenue en mélangeant au moins deux poudres métalliques d'alliage métallique.

8. Procédé selon l'une quelconque des revendications précédentes à 1 à 7, dans lequel ledit mélange de poudres métalliques a un rapport volumétrique entre les composants qui se trouve dans la plage entre 0,5 et 1,5.

9. Procédé selon la revendication précédente, dans lequel ledit rapport volumétrique est égal à 1.

10. Procédé selon l'une quelconque des revendications précédentes, **caractérisé en ce que** ledit liquide organique contient une substance comportant de 1 à 10 atomes de carbone.

11. Procédé selon la revendication précédente, **caractérisé en ce que** le liquide organique est sélectionné parmi les composés suivants : alcool éthylique, acétone, alcool méthylique, alcool propylique, éthyl-méthyl-cétone, diéthyl-cétone, acide acrylique, acide méthacrylique, diéthanolamine, diéthylamine, et analogue, et des mélanges de deux ou plus de deux desdites substances.

12. Procédé selon une ou plusieurs des revendications précédentes, **caractérisé en ce que** tout liquide organique en excès est éliminé par l'intermédiaire d'une permanence dans le four à une température inférieure à la température de frittage.

13. Procédé selon une ou plusieurs des revendications précédentes, **caractérisé en ce que** ladite poudre métallique est constituée d'un alliage contenant au moins 50 % de Pd.

14. Procédé selon une ou plusieurs des revendications précédentes 1 à 12, **caractérisé en ce que** la poudre métallique est constituée d'un alliage contenant au moins 50 % de Au.

15. Procédé selon une ou plusieurs des revendications précédentes 1 à 12, **caractérisé en ce que** la poudre métallique est constituée d'un alliage contenant au moins 50 % de Ag.

16. Procédé selon une ou plusieurs des revendications précédentes 1 à 12, **caractérisé en ce que** la poudre métallique est constituée d'or pur.

17. Procédé selon une ou plusieurs des revendications précédentes 1 à 12, **caractérisé en ce que** la poudre métallique est constituée d'argent pur.

18. Procédé selon une ou plusieurs des revendications précédentes, **caractérisé en ce que** ledit additif pour ladite poudre métallique est un composé organique sélectionné parmi les composés suivants :
polyéthylèneglycol, glycérine, caoutchouc butylique, caoutchouc isobutylique, caoutchouc naturel, cire naturelle, cire synthétique, et analogue et des mélanges de deux ou plus de deux desdites substances.

19. Procédé selon une ou plusieurs des revendications précédentes, **caractérisé en ce que** les dimensions des particules des poudres utilisées normalement se trouvent dans la plage entre 0,1 et 1 000 µm.
